# EUROPEAN PATENT SPECIFICATION

(11) **EP 4 486 272 B1**
(45) Date of publication and mention of the grant of the patent: **24.06.2026**
(21) Application number: 23710092.0
(22) Date of filing: 24.02.2023
(51) Int. Cl.: A61F 13/00, A61F 13/02

(54) **NEGATIVE PRESSURE WOUND THERAPY SYSTEM**
UNTERDRUCKWUNDTHERAPIESYSTEM
SYSTÈME DE TRAITEMENT DE PLAIE PAR PRESSION NÉGATIVE

(30) Priority: 01.03.2022 US 202263315385 P
(43) Date of publication of application: 08.01.2025
(73) Proprietor: Solventum Intellectual Properties Company, Eagan, MN 55121 (US)
(72) Inventor: MCGREGOR, Andrew J., Saint Paul, Minnesota 55133-3427 (US); KAZALA, Richard M., Jr., Saint Paul, Minnesota 55133-3427 (US); PRATT, Benjamin A., Bracknell Berkshire RG12 8HT (GB)
(74) Representative: Simmons & Simmons
(86) International application number: PCT/IB2023/051736
(87) International publication number: WO 2023/166392

(56) References cited:
- US-A1- 2020 179 581

## Description

### CROSS-REFERENCE TO RELATED APPLICATIONS

This application claims the benefit of priority to U.S. Provisional Application No. 63/315,385, filed on March 1, 2022.

### TECHNICAL FIELD

The invention set forth in the appended claims relates generally to tissue treatment systems and more particularly, but without limitation, to negative pressure wound therapy systems that use negative pressure to seal a dressing to skin surrounding a tissue site before treating the tissue site with negative pressure.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

While the clinical benefits of negative-pressure therapy are widely known, improvements to therapy systems, components, and processes may benefit healthcare providers and patients.

US 2020/179581 A1 relates to systems, methods, and apparatuses for treating a tissue site with reduced pressure involving a reduced-pressure interface having a multi-lumen conduit for contacting a manifold.

### BRIEF SUMMARY

The invention is defined by the independent claims. A selection of optional features of the invention is set out in the dependent claims.

Insofar as the term embodiment is used in the following, or features are presented as being optional, this should be interpreted in such a way that the only protection sought is that of the invention claimed (with due regard to Article 69 EPC and the protocol thereto).

References to "embodiment(s)" throughout the description which are not under the scope of the appended claims merely represent possible exemplary executions and are not part of the present invention.

### BRIEF DESCRIPTION OF THE DRAWINGS

Figure 1 is a functional block diagram of an example embodiment of a therapy system that can provide negative-pressure treatment in accordance with this specification;
Figure 2 is a partial sectional view of an illustrative embodiment of the therapy system of Figure 1 depicting an illustrative example embodiment of a dressing interface and a dressing deployed at a tissue site;
Figure 3 is an exploded view of a cover of the dressing of Figure 2;
Figure 4 is a sectional view of the cover of the dressing of Figure 2;
Figure 5 is a sectional view of the cover of the dressing of Figure 2 after application of a force to the cover;
Figure 6 is a perspective view of a tissue interface of the dressing of Figure 2;
Figure 7 is a sectional view of the dressing interface of Figure 2 with a first chamber in an open state;
Figure 8 is a sectional view of the dressing interface of Figure 2 with a second chamber in an open state;
Figure 9 is a sectional view of another example of the dressing interface of Figure 2;
Figure 10 is a sectional view of the therapy system of Figure 2 illustrating an operative embodiment of the therapy system delivering negative-pressure to a periphery of the dressing; and
Figure 11 is a sectional view of the therapy system of Figure 2 illustrating an operative embodiment of the therapy system delivering negative-pressure to the tissue site through a central portion of the dressing.

### DESCRIPTION OF EXAMPLE EMBODIMENTS

The following description of example embodiments provides information that enables a person skilled in the art to make and use the subject matter set forth in the appended claims, but it may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

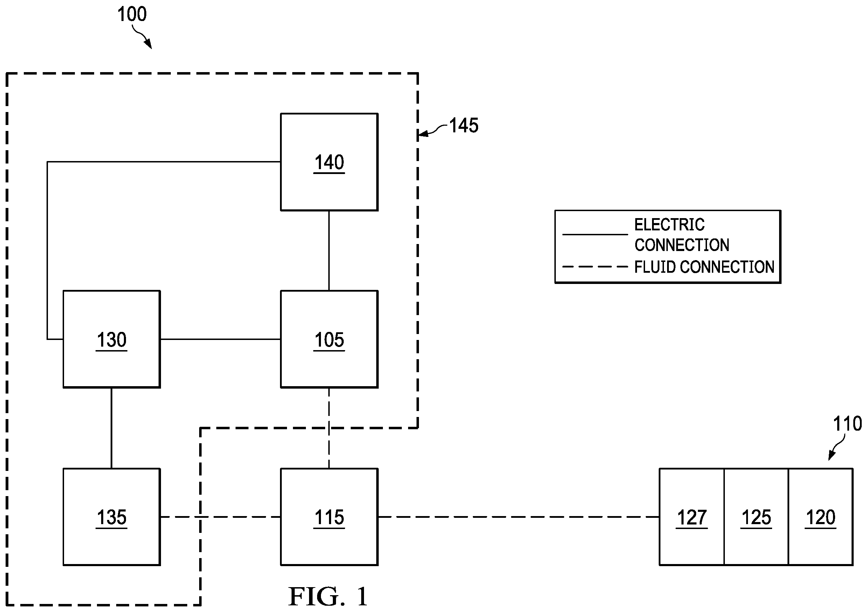

Figure 1 is a simplified functional block diagram of an example embodiment of a therapy system 100 that can provide negative-pressure therapy to a tissue site in accordance with this specification.

The term "tissue site" in this context broadly refers to a wound, defect, or other treatment target located on or within tissue, including, but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

The therapy system 100 may include a source or supply of negative pressure, such as a negative-pressure source 105, and one or more distribution components. A distribution component is preferably detachable and may be disposable, reusable, or recyclable. A dressing, such as a dressing 110, and a fluid container, such as a container 115, are examples of distribution components that may be associated with some examples of the therapy system 100. As illustrated in the example of Figure 1, the dressing 110 may comprise or consist essentially of a tissue interface 120, a cover 125, or both in some embodiments.

A fluid conductor is another illustrative example of a distribution component. A "fluid conductor," in this context, broadly includes a tube, pipe, hose, conduit, or other structure with one or more lumina or open pathways adapted to convey a fluid between two ends. Typically, a tube is an elongated, cylindrical structure with some flexibility, but the geometry and rigidity may vary. Moreover, some fluid conductors may be molded into or otherwise integrally combined with other components. Distribution components may also include or comprise interfaces or fluid ports to facilitate coupling and de-coupling other components. In some embodiments, for example, a dressing interface 127 may facilitate coupling a fluid conductor to the dressing 110. For example, the dressing interface 127 may be a SENSAT.R.A.C.^{™} Pad available from Kinetic Concepts, Inc. of San Antonio, Texas.

The therapy system 100 may also include a regulator or controller, such as a controller 130. Additionally, the therapy system 100 may include sensors to measure operating parameters and provide feedback signals to the controller 130 indicative of the operating parameters. As illustrated in Figure 1, for example, the therapy system 100 may include a first sensor 135 and a second sensor 140 coupled to the controller 130.

Some components of the therapy system 100 may be housed within or used in conjunction with other components, such as sensors, processing units, alarm indicators, memory, databases, software, display devices, or user interfaces that further facilitate therapy. For example, in some embodiments, the negative-pressure source 105 may be combined with the controller 130 and other components into a therapy unit 145.

In general, components of the therapy system 100 may be coupled directly or indirectly. For example, the negative-pressure source 105 may be directly coupled to the container 115 and may be indirectly coupled to the dressing 110 through the container 115. Coupling may include fluid, mechanical, thermal, electrical, or chemical coupling (such as a chemical bond), or some combination of coupling in some contexts. For example, the negative-pressure source 105 may be electrically coupled to the controller 130 and may be fluidly coupled to one or more distribution components to provide a fluid path to a tissue site. In some embodiments, components may also be coupled by virtue of physical proximity, being integral to a single structure, or being formed from the same piece of material.

A negative-pressure supply, such as the negative-pressure source 105, may be a reservoir of air at a negative pressure or may be a manual or electrically-powered device, such as a vacuum pump, a suction pump, a wall suction port available at many healthcare facilities, or a micro-pump, for example. "Negative pressure" generally refers to a pressure less than a local ambient pressure, such as the ambient pressure in a local environment external to a sealed therapeutic environment. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. References to increases in negative pressure typically refer to a decrease in absolute pressure, while decreases in negative pressure typically refer to an increase in absolute pressure. While the amount and nature of negative pressure provided by the negative-pressure source 105 may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -50 mm Hg (-6.7 kPa) and -300 mm Hg (-39.9 kPa).

The container 115 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

A controller, such as the controller 130, may be a microprocessor or computer programmed to operate one or more components of the therapy system 100, such as the negative-pressure source 105. In some embodiments, for example, the controller 130 may be a microcontroller, which generally comprises an integrated circuit containing a processor core and a memory programmed to directly or indirectly control one or more operating parameters of the therapy system 100. Operating parameters may include the power applied to the negative-pressure source 105, the pressure generated by the negative-pressure source 105, or the pressure distributed to the tissue interface 120, for example. The controller 130 is also preferably configured to receive one or more input signals, such as a feedback signal, and programmed to modify one or more operating parameters based on the input signals.

Sensors, such as the first sensor 135 and the second sensor 140, are generally known in the art as any apparatus operable to detect or measure a physical phenomenon or property, and generally provide a signal indicative of the phenomenon or property that is detected or measured. For example, the first sensor 135 and the second sensor 140 may be configured to measure one or more operating parameters of the therapy system 100. In some embodiments, the first sensor 135 may be a transducer configured to measure pressure in a pneumatic pathway and convert the measurement to a signal indicative of the pressure measured. In some embodiments, for example, the first sensor 135 may be a piezo-resistive strain gauge. The second sensor 140 may optionally measure operating parameters of the negative-pressure source 105, such as a voltage or current, in some embodiments. Preferably, the signals from the first sensor 135 and the second sensor 140 are suitable as an input signal to the controller 130, but some signal conditioning may be appropriate in some embodiments. For example, the signal may need to be filtered or amplified before it can be processed by the controller 130. Typically, the signal is an electrical signal, but may be represented in other forms, such as an optical signal.

The tissue interface 120 can be generally adapted to partially or fully contact a tissue site. The tissue interface 120 may take many forms, and may have many sizes, shapes, or thicknesses, depending on a variety of factors, such as the type of treatment being implemented or the nature and size of a tissue site. For example, the size and shape of the tissue interface 120 may be adapted to the contours of deep and irregular shaped tissue sites. Any or all of the surfaces of the tissue interface 120 may have an uneven, coarse, or jagged profile.

In some embodiments, the tissue interface 120 may comprise or consist essentially of a manifold. A manifold in this context may comprise or consist essentially of a means for collecting or distributing fluid across the tissue interface 120 under pressure. For example, a manifold may be adapted to receive negative pressure from a source and distribute negative pressure through multiple apertures across the tissue interface 120, which may have the effect of collecting fluid from across a tissue site and drawing the fluid toward the source. In some embodiments, the fluid path may be reversed or a secondary fluid path may be provided to facilitate delivering fluid across a tissue site.

In some illustrative embodiments, a manifold may comprise a plurality of pathways, which can be interconnected to improve distribution or collection of fluids. In some illustrative embodiments, a manifold may comprise or consist essentially of a porous material having interconnected fluid pathways. Examples of suitable porous material that can be adapted to form interconnected fluid pathways (e.g., channels) may include cellular foam, including open-cell foam such as reticulated foam; porous tissue collections; and other porous material such as gauze or felted mat that generally include pores, edges, and/or walls. Liquids, gels, and other foams may also include or be cured to include apertures and fluid pathways. In some embodiments, a manifold may additionally or alternatively comprise projections that form interconnected fluid pathways. For example, a manifold may be molded to provide surface projections that define interconnected fluid pathways.

In some embodiments, the tissue interface 120 may comprise or consist essentially of reticulated foam having pore sizes and free volume that may vary according to needs of a prescribed therapy. For example, reticulated foam having a free volume of at least 90% may be suitable for many therapy applications, and foam having an average pore size in a range of 400-600 microns (40-50 pores per inch) may be particularly suitable for some types of therapy. The tensile strength of the tissue interface 120 may also vary according to needs of a prescribed therapy. The 25% compression load deflection of the tissue interface 120 may be at least 0.35 pounds per square inch, and the 65% compression load deflection may be at least 0.43 pounds per square inch. In some embodiments, the tensile strength of the tissue interface 120 may be at least 10 pounds per square inch. The tissue interface 120 may have a tear strength of at least 2.5 pounds per inch. In some embodiments, the tissue interface may be foam comprised of polyols such as polyester or polyether, isocyanate such as toluene diisocyanate, and polymerization modifiers such as amines and tin compounds. In some examples, the tissue interface 120 may be reticulated polyurethane foam such as found in GRANUFOAM^{™} dressing or V.A.C. VERAFLO^{™} dressing, both available from Kinetic Concepts, Inc. of San Antonio, Texas.

The thickness of the tissue interface 120 may also vary according to needs of a prescribed therapy. For example, the thickness of the tissue interface may be decreased to reduce tension on peripheral tissue. The thickness of the tissue interface 120 can also affect the conformability of the tissue interface 120. In some embodiments, a thickness in a range of about 5 millimeters to 10 millimeters may be suitable.

The tissue interface 120 may be either hydrophobic or hydrophilic. In an example in which the tissue interface 120 may be hydrophilic, the tissue interface 120 may also wick fluid away from a tissue site, while continuing to distribute negative pressure to the tissue site. The wicking properties of the tissue interface 120 may draw fluid away from a tissue site by capillary flow or other wicking mechanisms. An example of a hydrophilic material that may be suitable is a polyvinyl alcohol, open-cell foam such as V.A.C. WHITEFOAM^{™} dressing available from Kinetic Concepts, Inc. of San Antonio, Texas. Other hydrophilic foams may include those made from polyether. Other foams that may exhibit hydrophilic characteristics include hydrophobic foams that have been treated or coated to provide hydrophilicity.

In some embodiments, the tissue interface 120 may be constructed from bioresorbable materials. Suitable bioresorbable materials may include, without limitation, a polymeric blend of polylactic acid (PLA) and polyglycolic acid (PGA). The polymeric blend may also include, without limitation, polycarbonates, polyfumarates, and capralactones. The tissue interface 120 may further serve as a scaffold for new cell-growth, or a scaffold material may be used in conjunction with the tissue interface 120 to promote cell-growth. A scaffold is generally a substance or structure used to enhance or promote the growth of cells or formation of tissue, such as a three-dimensional porous structure that provides a template for cell growth. Illustrative examples of scaffold materials include calcium phosphate, collagen, PLA/PGA, coral hydroxy apatites, carbonates, or processed allograft materials.

In some embodiments, the cover 125 may provide a bacterial barrier and protection from physical trauma. The cover 125 may also be constructed from a material that can reduce evaporative losses and provide a fluid seal between two components or two environments, such as between a therapeutic environment and a local external environment. The cover 125 may comprise or consist of, for example, an elastomeric film or membrane that can provide a seal adequate to maintain a negative pressure at a tissue site for a given negative-pressure source. The cover 125 may have a high moisture-vapor transmission rate (MVTR) in some applications. For example, the MVTR may be at least 250 grams per square meter per twenty-four hours in some embodiments, measured using an upright cup technique according to ASTM E96/E96M Upright Cup Method at 38°C and 10% relative humidity (RH). In some embodiments, an MVTR up to 5,000 grams per square meter per twenty-four hours may provide effective breathability and mechanical properties.

In some example embodiments, the cover 125 may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained. The cover 125 may comprise, for example, one or more of the following materials: polyurethane (PU), such as hydrophilic polyurethane; cellulosics; hydrophilic polyamides; polyvinyl alcohol; polyvinyl pyrrolidone; hydrophilic acrylics; silicones, such as hydrophilic silicone elastomers; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate (EVA); co-polyester; and polyether block polymide copolymers. Such materials are commercially available as, for example, Tegaderm^{®} drape, commercially available from 3M Company, Minneapolis Minnesota; polyurethane (PU) drape, commercially available from Avery Dennison Corporation, Pasadena, California; polyether block polyamide copolymer (PEBAX), for example, from Arkema S.A., Colombes, France; and Inspire 2301 and Inpsire 2327 polyurethane films, commercially available from Expopack Advanced Coatings, Wrexham, United Kingdom. In some embodiments, the cover 125 may comprise INSPIRE 2301 having an MVTR (upright cup technique) of 2600 g/m²/24 hours and a thickness of about 30 microns.

An attachment device may be used to attach the cover 125 to an attachment surface, such as undamaged epidermis, a gasket, or another cover. The attachment device may take many forms. For example, an attachment device may be a medically-acceptable, pressure-sensitive adhesive configured to bond the cover 125 to epidermis around a tissue site. In some embodiments, for example, some or all of the cover 125 may be coated with an adhesive, such as an acrylic adhesive, which may have a coating weight of about 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

In operation, the tissue interface 120 may be placed within, over, on, or otherwise proximate to a tissue site. If the tissue site is a wound, for example, the tissue interface 120 may partially or completely fill the wound, or it may be placed over the wound. The cover 125 may be placed over the tissue interface 120 and sealed to an attachment surface near a tissue site. For example, the cover 125 may be sealed to undamaged epidermis peripheral to a tissue site. Thus, the dressing 110 can provide a sealed therapeutic environment proximate to a tissue site, substantially isolated from the external environment, and the negative-pressure source 105 can reduce pressure in the sealed therapeutic environment.

The fluid mechanics of using a negative-pressure source to reduce pressure in another component or location, such as within a sealed therapeutic environment, can be mathematically complex. However, the basic principles of fluid mechanics applicable to negative-pressure therapy are generally well-known to those skilled in the art, and the process of reducing pressure may be described illustratively herein as "delivering," "distributing," or "generating" negative pressure, for example.

In general, exudate and other fluid flow toward lower pressure along a fluid path. Thus, the term "downstream" typically implies a position in a fluid path relatively closer to a source of negative pressure or further away from a source of positive pressure. Conversely, the term "upstream" implies a position relatively further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications, such as by substituting a positive-pressure source for a negative-pressure source, and this descriptive convention should not be construed as a limiting convention.

Negative pressure applied across the tissue site through the tissue interface 120 in the sealed therapeutic environment can induce macro-strain and micro-strain in the tissue site. Negative pressure can also remove exudate and other fluid from a tissue site, which can be collected in the container 115.

In some embodiments, the controller 130 may receive and process data from one or more sensors, such as the first sensor 135. The controller 130 may also control the operation of one or more components of the therapy system 100 to manage the pressure delivered to the tissue interface 120. In some embodiments, the controller 130 may include an input for receiving a desired target pressure and may be programmed for processing data relating to the setting and inputting of the target pressure to be applied to the tissue interface 120. In some example embodiments, the target pressure may be a fixed pressure value set by an operator as the target negative pressure desired for therapy at a tissue site and then provided as input to the controller 130. The target pressure may vary from tissue site to tissue site based on the type of tissue forming a tissue site, the type of injury or wound (if any), the medical condition of the patient, and the preference of the attending physician. After selecting a desired target pressure, the controller 130 can operate the negative-pressure source 105 in one or more control modes based on the target pressure and may receive feedback from one or more sensors to maintain the target pressure at the tissue interface 120.

Negative-pressure therapy has been repeatedly shown to be effective in the treatment of difficult to heal wounds. A challenge facing many medical professionals is creating an effective seal between a dressing of a negative-pressure therapy system and tissue, such as epidermis, surrounding a tissue site. Negative-pressure therapy may not be as effective if there are leaks or ways for fluid to escape from the dressing. A negative-pressure therapy system capable of being easily applied to a patient while also being effective at creating an air-tight seal is a long sought but unsolved solution in the art.

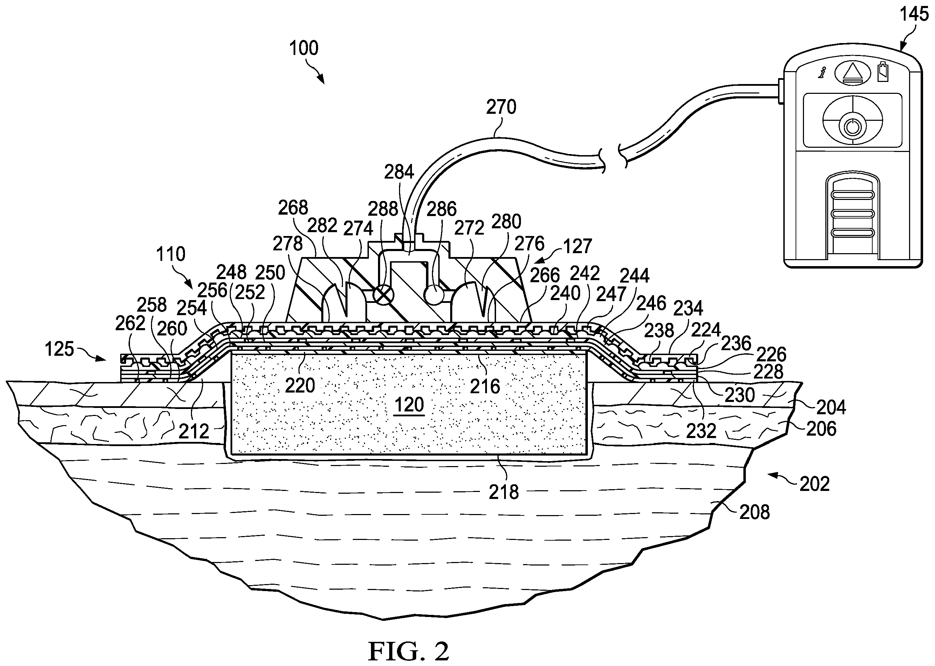

Figure 2 is a partial sectional view of an illustrative embodiment of the therapy system 100 of Figure 1 depicting an illustrative example embodiment of the dressing interface 127 and the dressing 110 deployed at a tissue site 202. The tissue site 202 may extend through or otherwise involve an epidermis 204, a dermis 206, and a subcutaneous tissue 208. The tissue site 202 may be a subsurface tissue site as depicted in Figure 2 that extends below the surface of the epidermis 204. Further, the tissue site 202 may be a surface tissue site (not shown) that predominantly resides on the surface of the epidermis 204, such as, for example, an incision. The therapy system 100 may provide therapy to, for example, the epidermis 204, the dermis 206, and the subcutaneous tissue 208, regardless of the positioning of the therapy system 100 or the type of tissue site. The therapy system 100 may also be utilized without limitation at other tissue sites.

In some embodiments, the tissue interface 120 may have a first surface 216 and a second surface 218 opposite the first surface 216. The second surface 218 can be disposed proximate to the tissue site 202. In some embodiments, a film 220 may be coupled to the first surface 216 of the tissue interface 120. The film 220 may cover the first surface 216 of the tissue interface 120 to fluidly isolate the tissue interface 120 from the cover 125. In other embodiments, the film 220 may extend over the tissue interface 120 to cover the entirety of the tissue interface 120 except for the second surface 218 of the tissue interface 120. The film 220 may be configured to isolate the tissue interface 120 from the cover 125 while allowing the tissue interface to be in fluid communication with the tissue site 202.

The film 220 may be a non-breathable film, membrane, or sheet that may be substantially vapor and liquid impermeable. The film 220 may comprise, for example, one or more of the following materials: hydrophilic polyurethane; cellulosics; hydrophilic polyamides; polyvinyl alcohol; polyvinyl pyrrolidone; hydrophilic acrylics; hydrophilic silicone elastomers; an INSPIRE 2301 material from Expopack Advanced Coatings of Wrexham, United Kingdom having, for example, an MVTR (inverted cup technique) of 14400 g/m²/24 hours and a thickness of about 30 microns; a thin, uncoated polymer drape; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; polyurethane (PU); EVA film; co-polyester; silicones; a silicone drape; a 3M Tegaderm^{®} drape; a polyurethane (PU) drape such as one available from Avery Dennison Corporation of Pasadena, California; polyether block polyamide copolymer (PEBAX), for example, from Arkema, France; Expopack 2327; or other appropriate material.

In some embodiments, the cover 125 may include multiple layers. For example, the cover 125 may include a stand-off layer 224, a microsphere layer 226, a film layer 228, a bonding adhesive layer 230, and a sealing adhesive layer 232. The stand-off layer 224 may include a first surface 234 and a second surface 236 opposite the first surface 234. The second surface 236 may include a plurality of surface features 238. The plurality of surface features 238 may extend from the second surface 236 of the stand-off layer 224 away from the first surface 234 of the stand-off layer 224. In some embodiments, the plurality of surface features 238 may be a plurality of projections. In some embodiments, the plurality of surface features 238 may be formed integrally with the stand-off layer 224. For example, the plurality of surface features 238 may be formed by embossing the stand-off layer 224. In other embodiments, the plurality of surface features 238 may be coupled to the second surface 236 of the stand-off layer 224. The plurality of surface features 238 may be coupled to the second surface 236 of the stand-off layer 224 by an adhesive, a weld, or another suitable method of securing the plurality of surface features 238 to the second surface 236 of the stand-off layer 224.

The stand-off layer 224 may be formed from a material that allows for a fluid seal. A fluid seal is a seal adequate to maintain negative pressure at a desired site given the particular negative pressure source or system involved. The stand-off layer 224 may comprise, for example, one or more of the following materials: hydrophilic polyurethane; cellulosics; hydrophilic polyamides; polyvinyl alcohol; polyvinyl pyrrolidone; hydrophilic acrylics; hydrophilic silicone elastomers; an INSPIRE 2301 material from Expopack Advanced Coatings of Wrexham, United Kingdom having, for example, an MVTR (inverted cup technique) of 14400 g/m²/24 hours and a thickness of about 30 microns; a thin, uncoated polymer drape; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; polyurethane (PU); EVA film; co-polyester; silicones; a silicone drape; a 3M Tegaderm^{®} drape; a polyurethane (PU) drape such as one available from Avery Dennison Corporation of Pasadena, California; polyether block polyamide copolymer (PEBAX), for example, from Arkema, France; Expopack 2327; or other appropriate material.

The stand-off layer 224 may be vapor permeable and liquid impermeable, thereby allowing vapor and inhibiting liquids from exiting the cover 125. In some embodiments, the stand-off layer 224 may be a flexible, breathable film, membrane, or sheet having a high MVTR of, for example, at least about 300g/m² per 24 hours. In other embodiments, a low or no vapor transfer drape might be used. The stand-off layer 224 may comprise a range of medically suitable films having a thickness between about 15 microns (µm) to about 50 microns (µm). In other embodiments, the stand-off layer 224 may be a non-breathable film, membrane, or sheet that may be substantially vapor and liquid impermeable.

The microsphere layer 226 may include a first surface 240 adjacent to the second surface 236 of the stand-off layer 224. The microsphere layer 226 may further include a second surface 242 opposite the first surface 240. In some embodiments, the microsphere layer 226 may include a plurality of microspheres 244 and an adhesive 246. The adhesive 246 may be disposed in a layer of material so that the adhesive 246 comprises the second surface 242 of the microsphere layer 226. The plurality of microspheres 244 may be disposed in a surface of the adhesive 246 opposite the second surface 242 so that the microspheres 244 and the adhesive 246 comprise the first surface 240. In some embodiments, the first surface 240 may be configured to have a first tack. The first surface 240 may further be configured to have a second tack after a force has been applied to the cover 125. The first tack of the microsphere layer 226 may be less than the second tack of the microsphere layer 226. A pathway 247 may be formed between the second surface 242 of the stand-off layer 224 and the first surface 240 of the microsphere layer 226. The pathway 247 may be configured to manifold fluid across the cover 125 and to prevent fluid flow in response to application of the force to the cover 125.

In some embodiments, each microsphere 244 of the plurality of microspheres 244 may be a substantially spherical particle. Each microsphere 244 may be partially submerged in the adhesive 246 so that at least a portion of the microsphere 244 may protrude from the adhesive 246 on the first surface 240. Each microsphere 244 of the plurality of microspheres 244 may be separated from an adjacent microsphere 244 by about 0.1mm to 0.5mm. For example, an edge of one of the microspheres 244 may be separated from an adjacent edge of an adjacent microsphere 244 by about 0.1mm to 0.5mm. Preferably, each microsphere 244 may be separated from an adjacent microsphere 244 by about 0.2mm to 0.4mm. In some embodiments, the adhesive 246 may include a plurality of depressions configured to receive the plurality of microspheres 244. In some embodiments, each microsphere may have an average effective diameter between about 25µm and about 1500 µm . In other embodiments, the plurality of microspheres 244 may be sized and spaced differently in order to provide the first tack and the second tack to the microsphere layer 226. An average effective diameter of a body may be a diameter of a circular or spherical body having the same area or volume as the area or volume of the non-circular or non-spherical body. After application of a force to the cover 125, the plurality of microspheres 244 may be configured to be embedded into the adhesive 246 so that the adhesive 246 substantially surrounds the plurality of microspheres 244. In some embodiments, the plurality of microspheres 244 may be comprised of a material having sufficient rigidity and structural integrity to withstand the negative-pressure required for negative-pressure treatment. For example, in some embodiments, the plurality of microspheres 244 may be formed from an acrylic or a glass material. In other embodiments, the plurality of microspheres 244 may include a plurality of particles. Each particle may be acrylic or glass or another material that is configured to protrude from the adhesive 246 of the first surface 240 of the microsphere layer 226.

The adhesive 246 may be a medically-acceptable adhesive. The adhesive 246 may also be flowable. For example, the adhesive 246 may comprise an acrylic adhesive, rubber adhesive, high-tack silicone adhesive, polyurethane, or other adhesive substance. In some embodiments, the adhesive 246 may be a pressure-sensitive adhesive comprising an acrylic adhesive with coating weight of about 15 grams/m² (gsm) to about 70 grams/m² (gsm). In other embodiments, the adhesive 246 may have a coating weight of less than 15 grams/m² (gsm) or greater than 70 grams/m² (gsm) depending on the desired first tack and second tack of the first surface 240 of the microsphere layer 226.

The film layer 228 may have a first surface 248 adjacent to the second surface 242 of the microsphere layer 226. The film layer 228 may have a second surface 250 opposite the first surface 248. In some embodiments, the film layer 228 may include a plurality of perforations 252. The film layer 228 may be formed from a material that allows for a fluid seal. A fluid seal is a seal adequate to maintain negative pressure at a desired site given the particular negative pressure source or system involved. The film layer 228 may comprise, for example, one or more of the following materials: hydrophilic polyurethane; cellulosics; hydrophilic polyamides; polyvinyl alcohol; polyvinyl pyrrolidone; hydrophilic acrylics; hydrophilic silicone elastomers; an INSPIRE 2301 material from Expopack Advanced Coatings of Wrexham, United Kingdom having, for example, an MVTR (inverted cup technique) of 14400 g/m²/24 hours and a thickness of about 30 microns; a thin, uncoated polymer drape; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; polyurethane (PU); EVA film; co-polyester; silicones; a silicone drape; a 3M Tegaderm^{®} drape; a polyurethane (PU) drape such as one available from Avery Dennison Corporation of Pasadena, California; polyether block polyamide copolymer (PEBAX), for example, from Arkema, France; Expopack 2327; or other appropriate material.

In some embodiments, the film layer 228 may be vapor permeable and liquid impermeable and may be a flexible, breathable film, membrane, or sheet having a high MVTR of, for example, at least about 300g/m² per 24 hours. In other embodiments, a low or no vapor transfer drape might be used. The film layer 228 may comprise a range of medically suitable films having a thickness between about 15 microns (µm) to about 50 microns (µm). In other embodiments, the film layer 228 may be a non-breathable film, membrane, or sheet that may be substantially vapor and liquid impermeable.

The bonding adhesive layer 230 may have a first surface 254 adjacent to the second surface 250 of the film layer 228. The bonding adhesive layer 230 may have a second surface 256 opposite the first surface 254. In some embodiments, the bonding adhesive layer 230 may be a patterned adhesive forming a plurality of perforations through the bonding adhesive layer 230. In other embodiments, the bonding adhesive layer 230 may be a continuous layer having a plurality of perforations formed therein or may be a continuous layer that is substantially free of openings. The bonding adhesive layer 230 may be configured to have a first bond strength. The bonding adhesive layer 230 may be a medically-acceptable adhesive. The bonding adhesive layer 230 may also be flowable. For example, the bonding adhesive layer 230 may comprise an acrylic adhesive, rubber adhesive, high-tack silicone adhesive, polyurethane, or other adhesive substance. In some embodiments, the bonding adhesive layer 230 may be a pressure-sensitive adhesive comprising an acrylic adhesive with coating weight of about 4 grams/m² (gsm) to about 70 grams/m² (gsm). In other embodiments, the bonding adhesive layer 230 may have a coating weight of less than 15 grams/m² (gsm) or greater than 70 grams/m² (gsm) depending on the desired first bond strength of the bonding adhesive layer 230. The bonding adhesive layer 230 may have a thickness of about 30 micrometers to about 40 micrometers in some embodiments. In other embodiments, the bonding adhesive layer 230 may have a thickness less than 30 micrometers or greater than 40 micrometers.

The sealing adhesive layer 232 may have a first surface 258 adjacent to the second surface 256 of the bonding adhesive layer 230. The sealing adhesive layer 232 may have a second surface 260 opposite the first surface 258. In some embodiments, the sealing adhesive layer 232 may have a plurality of openings 262. In other embodiments, the sealing adhesive layer 232 may be a patterned adhesive. In embodiments where the sealing adhesive layer 232 is a patterned adhesive, the sealing adhesive layer 232 may be pattern coated with a pattern that provides an amount of adhesive that gives a desired bond strength. For example, the sealing adhesive layer 232 may be configured to have a second bond strength. The second bond strength of the sealing adhesive layer 232 may be less than the first bond strength of the bonding adhesive layer 230. The sealing adhesive layer 232 may be a medically-acceptable adhesive. The sealing adhesive layer 232 may also be flowable. For example, the sealing adhesive layer 232 may comprise an acrylic adhesive, rubber adhesive, high-tack silicone adhesive, polyurethane, or other adhesive substance. In some embodiments, the sealing adhesive layer 232 may be a pressure-sensitive adhesive comprising an acrylic adhesive with coating weight of about 4 grams/m² (gsm) to about 70 grams/m² (gsm). In other embodiments, the sealing adhesive layer 232 may have a coating weight of less than 4 grams/m² (gsm) or greater than 70 grams/m² (gsm) depending on the desired second bond strength of the sealing adhesive layer 232. The sealing adhesive layer 232 may have a thickness of about 30 micrometers to about 40 micrometers in some embodiments. In other embodiments, the sealing adhesive layer 232 may have a thickness less than 30 micrometers or greater than 40 micrometers.

The therapy system 100 may further include a dressing connector, or the dressing interface 127. The dressing interface 127 may have a first surface or a first end 266 and a second surface or a second end 268. The first end 266 may be coupled to the cover 125 and the second end may be coupled to a conduit 270 that may enable fluid communication between the therapy unit 145 and the dressing 110. The dressing interface 127 may include a first chamber 272 and a second chamber 274. The first end 266 may have a first opening 276 that may expose the first chamber 272 to the first surface 234 of the stand-off layer 224. The first end 266 may also have a second opening 278 that may expose the second chamber 274 to the first surface 234 of the stand-off layer 224. The dressing interface 127 may also include a first piercing element 280 disposed in the first chamber 272 and a second piercing element 282 disposed in the second chamber 274.

The dressing interface 127 may further include a fluid pathway 284, a first valve 286, and a second valve 288. The first valve 286 may fluidly couple the fluid pathway 284 to the first chamber 272 and the second valve 288 may fluidly couple the fluid pathway 284 to the second chamber 274. The first valve 286 and the second valve 288 may be a type of valve configured to provide a fluid seal to the first chamber 272 and the second chamber 274. In some embodiments, the first valve 286 and the second valve 288 may be check valves. Exemplary check valves may include ball check valves, diaphragm check valves, flap-style check valves, swing check valves, stop-check valves, duckbill valves, pneumatic non-return valves, or other one-way valves configured to automatically permit fluid flow in a single direction and to prevent fluid flow in any other direction. In other embodiments, the first valve 286 and the second valve 288 may be a different type of valve that can permit and restrict fluid flow and can withstand the application of negative pressure. Both the first valve 286 and the second valve 288 may be configured to be actuated either manually or automatically.

In some embodiments, there may be one or more attachment points that are configured to secure the dressing interface 127 to the cover 125. The dressing interface 127 may be coupled to the dressing with adhesive or any other method of coupling that is configured to secure the dressing interface 127 in place at the dressing 110 during the application of negative pressure.

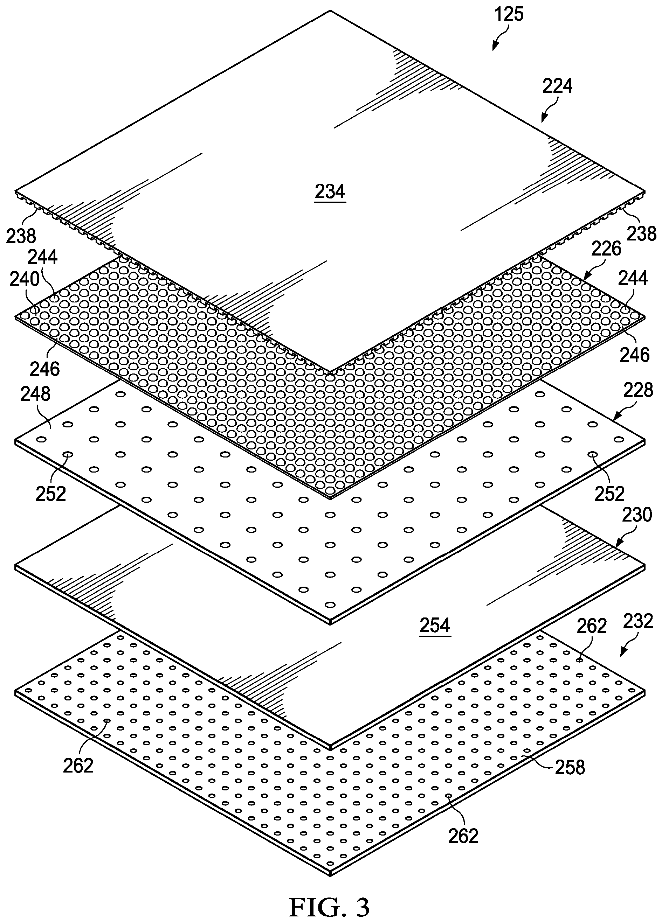

Figure 3 is an exploded view of the cover 125 of Figure 2. In some embodiments, the cover 125 may be square. In other embodiments, the cover 125 may be other shapes and may be cut to shape to fit the tissue site 202. The stand-off layer 224 may be positioned adjacent to the first surface 240 of the microsphere layer 226 such that the second surface 236 of the stand-off layer 224 is adjacent to the first surface 240 of the microsphere layer 226. The plurality of surface features 238 of the stand-off layer 224 may extend from the second surface 23 6 of the stand-off layer 224 towards the first surface 240 of the microsphere layer 226.

The microsphere layer 226 may be positioned between the stand-off layer 224 and the film layer 228 such that the first surface 240 of the microsphere layer 226 is adjacent to the second surface 236 of the stand-off layer and the second surface 242 of the microsphere layer 226 is adjacent to the first surface 248 of the film layer 228. The plurality of microspheres 244 may extend from the first surface 240 of the microsphere layer 226 towards the second surface 236 of the stand-off layer 224. In some embodiments, the plurality of microspheres 244 may be distributed uniformly across the first surface 240 of the microsphere layer 226. In other embodiments, the plurality of microspheres 244 may be distributed irregularly across the first surface 240 of the microsphere layer 226. Each microsphere 244 of the plurality of microspheres 244 may be separated from every other microsphere 244 of the plurality of microspheres 244. The adhesive 246 may be exposed to the second surface 236 of the stand-off layer 224 between each microsphere 244 of the plurality of microspheres 244.

The film layer 228 may be positioned between the microsphere layer 226 and the bonding adhesive layer 230 such that the first surface 248 of the film layer 228 is adjacent to the second surface 242 of the microsphere layer 226 and the second surface 250 of the film layer 228 is adjacent to the first surface 254 of the bonding adhesive layer 230. In some embodiments, the plurality of perforations 252 of the film layer 228 may be circular openings as shown in Figure 3. In other embodiments, the plurality of perforations 252 may be slits or openings of a different size or shape that are configured to provide fluid communication through the film layer 228. In some embodiments, the plurality of perforations 252 may be distributed uniformly across the first surface 248 of the film layer 228. The plurality of perforations 252 may have a pitch between about 1mm and about 10mm in some embodiments. In other embodiments, the plurality of perforations 252 may be distributed irregularly across the first surface 248 of the film layer 228.

The bonding adhesive layer 230 may be positioned between the film layer 228 and the sealing adhesive layer 232 such that the first surface 254 of the bonding adhesive layer 230 is adjacent to the second surface 250 of the film layer and the second surface 256 of the bonding adhesive layer 230 is adjacent to the first surface 258 of the sealing adhesive layer 232. As shown in Figure 3, the bonding adhesive layer 230 may be a continuous layer of adhesive. In other embodiments, the bonding adhesive layer 230 may be a patterned adhesive or there may be a plurality of perforations through the bonding adhesive layer 230.

The sealing adhesive layer 232 may be positioned adjacent to the bonding adhesive layer 230 such that the first surface 258 of the sealing adhesive layer 232 is adjacent to the second surface 256 of the bonding adhesive layer 230. The sealing adhesive layer 232 may include the plurality of openings 262. In some embodiments, the plurality of openings 262 may be circular as shown in Figure 3. The plurality of openings 262 may each have a diameter between about 0.01mm and about 5mm in some embodiments. In other embodiments, the plurality of openings 262 may be openings of a different size or shape that enable fluid communication through the sealing adhesive layer 232. In some embodiments, the plurality of openings 262 may be distributed uniformly across the first surface 258 of the sealing adhesive layer 232. The plurality of openings 262 may have a pitch between about 1mm to about 10mm in some embodiments. In other embodiments, the plurality of openings 262 may be distributed irregularly across the first surface 258 of the sealing adhesive layer 232. In some embodiments, the plurality of openings 262 of the sealing adhesive layer 232 may be misaligned with the plurality of perforations 252 of the film layer 228 and in other embodiments, the plurality of openings 262 of the sealing adhesive layer 232 may be substantially aligned with the plurality of perforations 252 of the film layer 228.

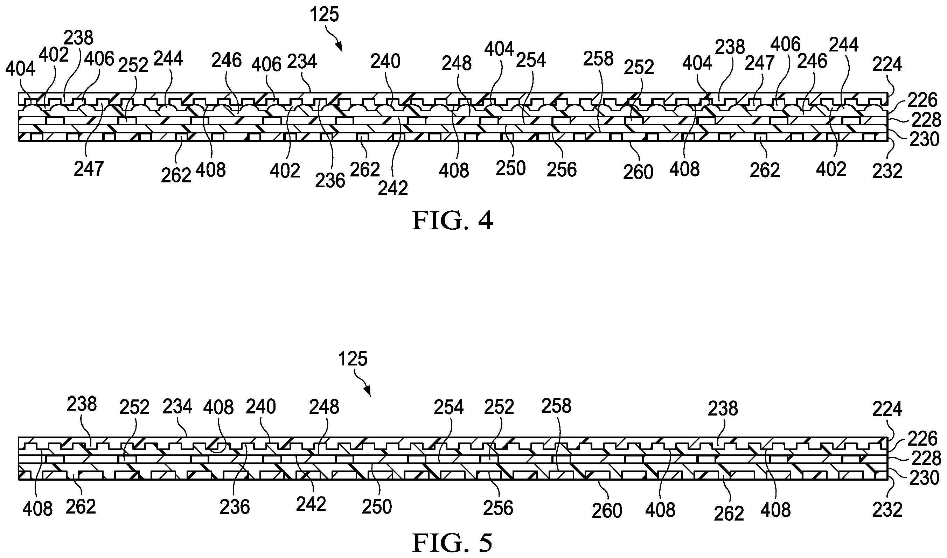

Figure 4 is a sectional view of the cover 125 of Figures 2 and 3. The microsphere layer 226, the film layer 228, and the bonding adhesive layer 230 are disposed between the stand-off layer 224 and the sealing adhesive layer 232. The plurality of microspheres 244 of the microsphere layer 226 may extend away from the first surface 240 of the microsphere layer 226 towards the second surface 236 of the stand-off layer 224. There may be a space 402 between the first surface 240 of the microsphere layer 226 and a top 404 of each microsphere 244 of the plurality of microspheres 244. The plurality of surface features 238 of the stand-off layer 224 may extend away from the second surface 236 of the stand-off layer 224 towards the first surface 240 of the microsphere layer 226. There may be a space 406 between the second surface 236 of the stand-off layer 224 and an end 408 of each surface feature 238 of the plurality of surface features 238. The plurality of surface features 238 of the stand-off layer 224 may contact at least some of the plurality of microspheres 244 of the microsphere layer 226. The space 402 may intersect with the space 406 to form the pathway 247 such that fluid can flow between the second surface 236 of the stand-off layer 224 and the first surface 240 of the microsphere layer 226.

Figure 4 may show the cover 125 before a force, such as negative pressure from the negative-pressure source 105, has been applied to the cover 125. Prior to the application of a force, the adhesive 246 may provide the first tack to the first surface 240 of the microsphere layer 226 and a second tack to the second surface 242 of the microsphere layer 226. The second tack of the second surface 242 of the microsphere layer 226 may adhere the microsphere layer 226 to the film layer 228. The plurality of microspheres 244 of the microsphere layer 226 may not be embedded into the adhesive 246 of the microsphere layer 226 before the force is applied to the cover 125. At least a portion of a surface are of the first surface 240 comprises the microspheres 244. The microspheres 244 may be generally non-adherent so that only those portions of the first surface 240 that comprise the adhesive 246 may be free to adhere to another body, such as the second surface 236 of the stand-off layer 224. In some embodiments, at least a portion of the ends 408 of the plurality of surface features 238 may contact the adhesive 246 between the microspheres 244, giving the first surface 240 the first tack. Preferably, at least a portion of the ends 408 of the plurality of surface features 238 may contact the adhesive 246 while maintaining the pathway 247.

If the cover 125 is positioned at the tissue site 202, the cover 125 can be held in place by the second bond strength of the sealing adhesive layer 232.The bonding adhesive layer 230 may be separated from the tissue site 202 prior to application of the force to the cover 125, and contact between the tissue site 202 and the cover 125 may be between the tissue site 202 and the sealing adhesive layer 232.

Figure 5 is a sectional view of the cover 125 of Figures 2-4, following the application of a force to the cover 125. In some embodiments, the force applied to the cover 125 may be negative pressure from the negative-pressure source 105. In other embodiments, the force may be another external factor that applies pressure to the cover 125. If a force is applied to the cover 125, the layers of the cover 125 may be pressed together. For example, if the force is negative pressure from the negative-pressure source 105, tissue surrounding the tissue site 202 may be pulled into the plurality of openings 262 of the sealing adhesive layer 232 to contact the bonding adhesive layer 230. If the force is a source other than negative pressure from the negative-pressure source 105, the bonding adhesive layer 230 may be pushed through the plurality of openings 262 of the sealing adhesive layer 232. After the force is applied the cover 125, the tissue surrounding the tissue site 202 may be in contact with the bonding adhesive layer 230. The bonding adhesive layer 230 may hold the cover 125 in place at the tissue surrounding the tissue site 202 with the first bond strength, which may be greater than the second bond strength from the sealing adhesive layer 232.

After the tissue surrounding the tissue site 202 is coupled to the bonding adhesive layer 230, the microsphere layer 226 may be pulled towards the stand-off layer 224. The plurality of microspheres 244 may be embedded into the adhesive 246 to eliminate the space 402. For example, negative pressure may be supplied to the pathway 247. The application of negative pressure to the pathway 247 may cause the pathway 247 to collapse and draw the adhesive 246 into the space 402 and the space 406. Drawing the adhesive into the space 402 and the space 406 may cause the adhesive to surround the microspheres 244, embedding the microspheres 244 in the adhesive 246. The microsphere layer 226 may also be pulled into the stand-off layer 224 to eliminate the space 406. Once the space 402 and the space 406 are eliminated, the pathway 247 may be eliminated and fluid can no longer flow between the stand-off layer 224 and the microsphere layer 226.

After the force is applied to the cover 125 and the plurality of microspheres 244 are embedded into the adhesive 246, the microsphere layer 226 may be coupled to the stand-off layer 224 with the second tack. The microsphere layer 226 may have the second tack because the plurality of microspheres 244 may be embedded into the adhesive 246 and a greater amount of the adhesive 246 may be exposed to the stand-off layer 224. If a greater amount of the adhesive 246 is exposed to the stand-off layer 224, the second tack coupling the microsphere layer 226 and the stand-off layer 224 may be greater than the first tack and may adhesively couple the microsphere layer 226 and the stand-off layer 224 together. Once the microsphere layer 226 is coupled to the stand-off layer 224 with the second tack, the bond between the microsphere layer 226 and the stand-off layer 224 may be equal to the bond between the microsphere layer 226 and the film layer 228.

In other embodiments, the plurality of microspheres 244 may be disposed on the second surface 242 of the microsphere layer 226. The adhesive 246 may be in contact with the stand-off layer 224 with the second tack and the microsphere layer 226 may contact the film layer 228 with the first tack prior to a force being applied to the cover 125. When a force is applied to the cover 125, the bonding adhesive layer 230 may contact the tissue surrounding the tissue site 202 and the plurality of microspheres 244 may be embedded into the adhesive 246 substantially as described above. The microsphere layer 226 may then be coupled to both the stand-off layer 224 and the film layer 228 with the second tack.

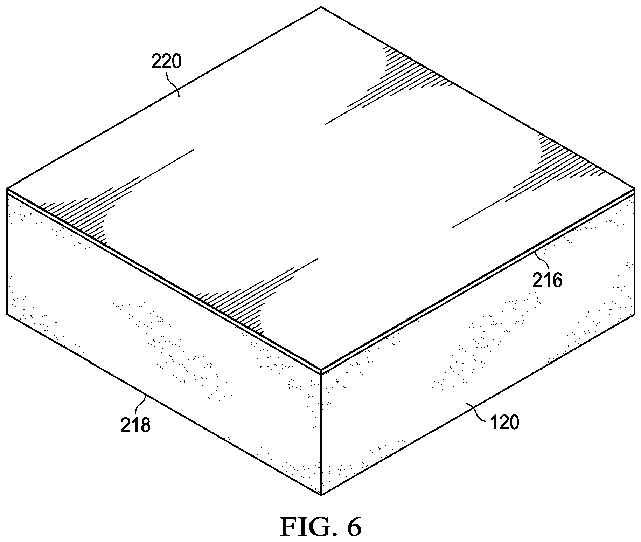

Figure 6 is a perspective view of the tissue interface 120 of the dressing 110. The tissue interface 120 may include the film 220. The film 220 may be coupled to the first surface 216 of the tissue interface 120 such that it covers the first surface 216 of the tissue interface 120. The film 220 may isolate the tissue interface 120 from the cover 125 and may allow fluid communication between the cover 125 and the tissue interface 120 if the film 220 is pierced. The tissue interface 120 appears as a rectangular block. In other embodiments, the tissue interface 120 may be cut to fit a specific wound or tissue site or may be manufactured in other shapes or sizes.

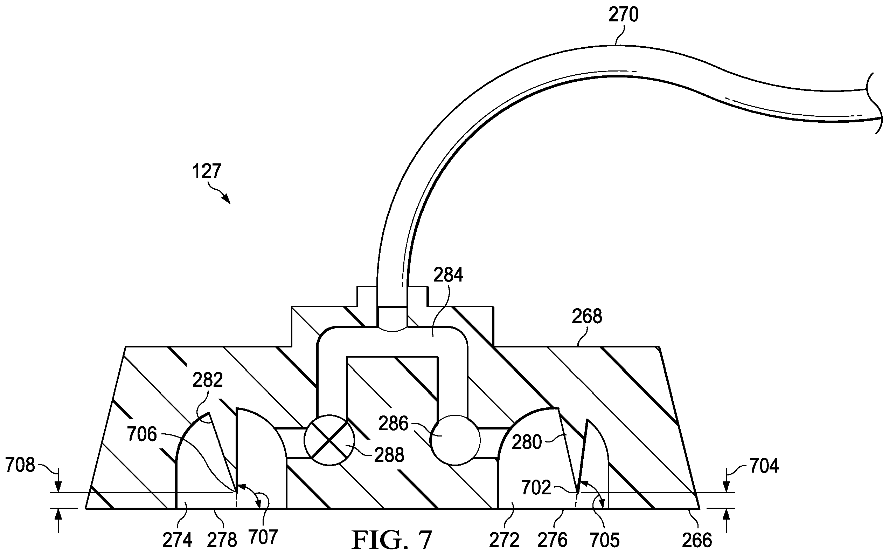

Figure 7 is a sectional view of the dressing interface 127 of Figure 2. The dressing interface 127 may include the first chamber 272 and the second chamber 274. The negative-pressure source 105 may be fluidly coupled to the dressing interface 127 through the conduit 270. In Figure 7, the second valve 288 is closed and the first valve 286 is open. The fluid pathway 284 may fluidly couple the conduit 270 to the first chamber 272. The fluid pathway 284 may be isolated from the second chamber 274 by the closure of the second valve 288. If the negative-pressure source 105 is actuated, the negative-pressure source 105 may draw fluid from the first chamber 272 to develop a negative pressure in the first chamber 272. The second chamber 274, being isolated from the fluid pathway 284 may not be in fluid communication with the negative-pressure source 105.

The first chamber 272 may include the first piercing element 280 and the second chamber 274 may include the second piercing element 282. The first piercing element 280 may have a first piercing end 702. The first piercing end 702 may be a first distance 704 away from the first opening 276. The first piercing element 280 may be disposed at a first angle 705 relative to the first end 266 of the dressing interface 127. In some embodiments, the first angle 705 may be between about 30 and 90 degrees.

The second piercing element 282 may have a second piercing end 706. The second piercing end 706 may be a second distance 708 away from the second opening 278. The second piercing element 282 may be disposed at a second angle 707 relative to the second end 268 of the dressing interface 127. In some embodiments, the second angle 707 may be between about 30 and 90 degrees. In some embodiments, the first distance 704 may be different from the second distance 708. For example, the first distance 704 may be greater than the second distance 708. In some embodiments, the first angle 705 may be different from the second angle 707.

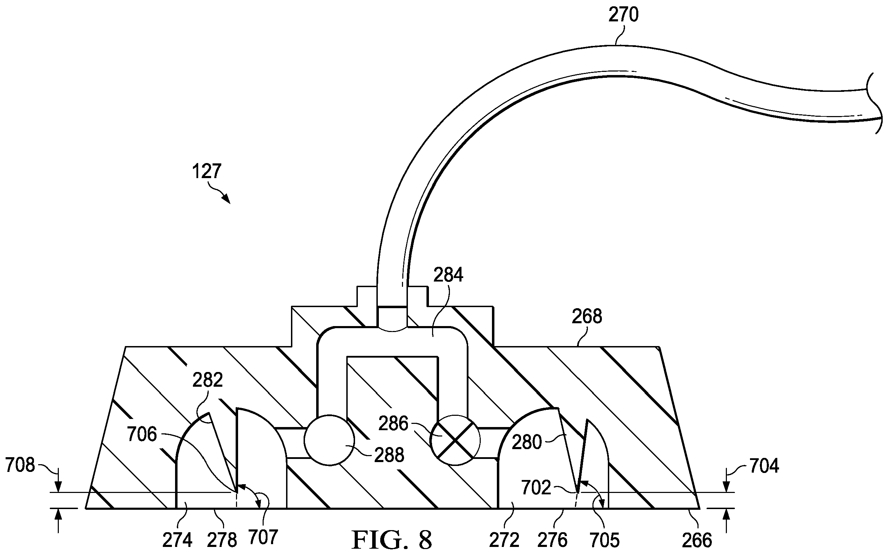

Figure 8 is a sectional view of the dressing interface 127 of Figures 2 and 7 having the first valve 286 closed and the second valve 288 open. The fluid pathway 284 may fluidly couple the conduit 270 to the second chamber 274, and because the first valve 286 is closed, the fluid pathway 284 may be isolated from the first chamber 272. If the negative-pressure source 105 is actuated, the negative-pressure source 105 may draw fluid from the second chamber 274, developing a negative pressure in the second chamber 274. The first chamber 272, being fluidly isolated by the first valve 286, may not be in fluid communication with the negative-pressure source 105.

Referring to Figures 7 and 8, in some embodiments, the dressing interface 127 may be configured to draw fluid from the first chamber 272 at a first predetermined pressure and to draw fluid from the second chamber 274 at a second predetermined pressure. For example, the first valve 286 can be configured to be closed at ambient pressure and can be actuated in response to the first predetermined pressure. When the first valve 286 is actuated, the first valve 286 may allow the first chamber 272 to be fluidly coupled to the conduit 270 and the negative-pressure source 105. The second valve 288 can be configured to be closed at ambient pressure and can be actuated in response to the second predetermined pressure. When the second valve 288 is actuated, the second valve 288 may allow the second chamber 274 to be fluidly coupled to the conduit 270 and the negative-pressure source 105. In some example embodiments, the first predetermined pressure may be at or around -150mmHg and the second predetermined pressure may be at or around -175mmHg. In other embodiments, the first predetermined pressure and the second predetermined pressure may be different values depending on the desired therapy being delivered to the tissue site 202.

In some embodiments, the first valve 286 may be open at ambient pressure and may be in fluid communication with the negative-pressure source 105 until the negative-pressure source 105 begins drawing fluid at a threshold pressure. At the threshold pressure, the first valve 286 may close and the second valve 288 may open. In other embodiments, the first valve 286 and the second valve 288 may both be closed at ambient pressure. The first valve 286 may open at the first predetermined pressure, and the second valve 288 may open at the second predetermined pressure. In some embodiments, the first valve 286 may remain open when the second valve 288 opens at the second predetermined pressure. In any of the above embodiments, the first valve 286 and the second valve 288 may be communicatively coupled to the controller 130 of the therapy unit 145. The controller 130 may be configured to actuate the first valve 286 and the second valve 288 in response to at least the threshold pressure, the first predetermined pressure, and the second predetermined pressure. In other embodiments, the first valve 286 and the second valve 288 may be manually actuated by a patient or a health care provider in response to the pressure being delivered to the dressing interface 127 by the negative-pressure source 105.

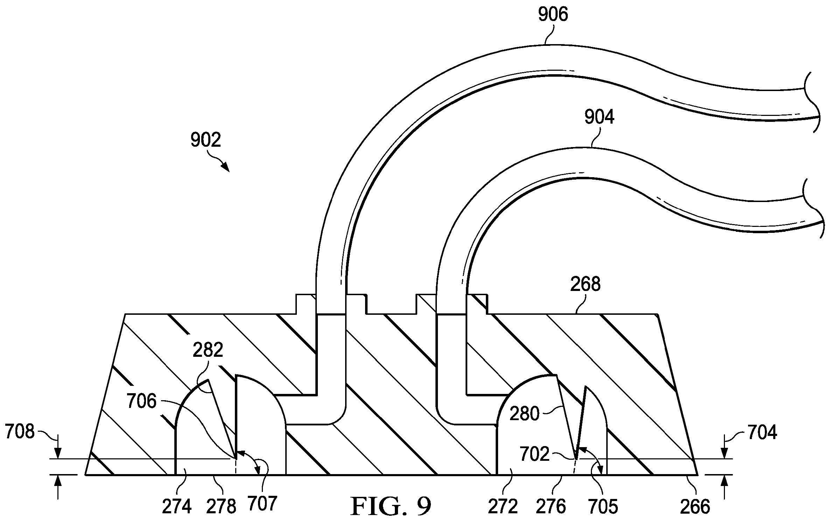

Figure 9 is a sectional view of another example embodiment of a dressing interface 902 that can be used with the therapy system 100. The dressing interface 902 may have the first chamber 272, the second chamber 274, the first piercing element 280, and the second piercing element 282 substantially as described above with reference to the dressing interface 127. The dressing interface 902 may have a first conduit 904 and a second conduit 906. The first conduit 904 may fluidly couple the negative-pressure source 105 to the first chamber 272, and the second conduit 906 may fluidly couple the negative-pressure source 105 to the second chamber 274.

In some embodiments, the negative-pressure source 105 may be configured to draw fluid from the first chamber 272 at the first predetermined pressure and to draw fluid from the second chamber 274 at the second predetermined pressure. The negative-pressure source 105 may be fluidly connected to both the first conduit 904 and the second conduit 906. The negative-pressure source 105 may be configured to deliver negative pressure through the first conduit 904 to the first chamber 272 at the first predetermined pressure. The negative-pressure source 105 may be configured to deliver negative pressure through the second conduit 906 to the second chamber 274 at the second predetermined pressure. In other embodiments, the negative-pressure source 105 may be configured to deliver negative pressure to the first chamber 272 from ambient pressure to the threshold pressure. At the threshold pressure, the negative-pressure source 105 may stop delivering negative pressure through the first conduit 904 and may start delivering negative pressure through the second conduit 906 to the second chamber 274. In still other embodiments, the negative-pressure source 105 may deliver pressure through the first conduit 904 until the threshold pressure and then may deliver pressure through both the first conduit 904 and the second conduit 906 after the threshold pressure.

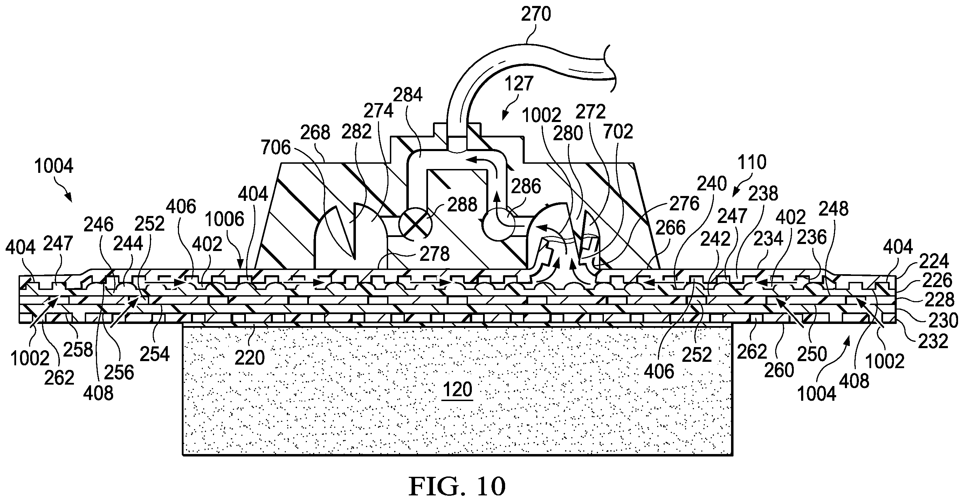

Figure 10 is a sectional view of the dressing 110 and the dressing interface 127 of the therapy system 100. The first valve 286 of the dressing interface 127 may be open and may fluidly couple the first chamber 272 to the negative-pressure source 105 through the conduit 270. The cover 125 may have a periphery 1004 that may surround a central portion 1006 of the cover 125. The central portion 1006 of the cover 125 may be the area of the cover 125 aligned with the tissue interface 120. The periphery 1004 of the cover 125 may be the area of the cover 125 that is not in contact with the tissue interface 120. Before the negative-pressure source 105 is actuated, the layers of the cover 125 in the periphery 1004 of the cover 125 may be sealed or pressed together. In some embodiments, a user or a health care provider may apply a force to the periphery 1004 of the cover 125 to embed the layers together as discussed above with reference to Figure 5. In other embodiments, layers of the cover 125 in the periphery 1004 of the cover 125 may be sealed together by the shearing action of a die cut or a scissor cut. For example, if the cover 125 was cut to shape to fit the tissue site 202 before being applied to the tissue site 202, the layers of the cover 125 in the periphery 1004 may be coupled together as discussed above with reference to Figure 5 before the cover 125 is applied to the tissue site 202. The negative-pressure source 105 may be operating at the first predetermined pressure and may be generate a fluid flow 1002 from the first chamber 272.

The stand-off layer 224 may be pulled away from the microsphere layer 226 and into the first chamber 272 by the negative-pressure source 105. The first piercing element 280 and the second piercing element 282 may be located at different angles within the first chamber 272 and the second chamber 274, respectively, to ensure that the proper elements of the dressing 110 are pierced when the therapy system 100 is operating. For example, the first angle 705 of the first piercing element may ensure that only the stand-off layer 224 is pierced by the first piercing element 280. The microsphere layer 226, being coupled to the stand-off layer 224 by the first tack and to the film layer 228 with the second tack, may not pulled into the first chamber 272. The first piercing end 702 of the first piercing element 280 may pierce through the stand-off layer 224. The negative-pressure source 105 may generate a fluid flow through the pathway 247 between the stand-off layer 224 and the microsphere layer 226. The fluid flow 1002 may be drawn through the cover 125 from the periphery 1004 of the cover 125. There may be no fluid flow from the tissue interface 120 or the central portion 1006 of the cover 125 because the microsphere layer 226 and the film 220 of the tissue interface 120 have not been pierced by the first piercing element 280.

The negative-pressure source 105 may generate a force on the tissue surrounding the tissue site 202 through the layers of the cover 125 and may pull the tissue surrounding the tissue site 202 into the plurality of openings 262 of the sealing adhesive layer 232. The tissue surrounding the tissue site 202 may then be in contact with the bonding adhesive layer 230 and if the negative-pressure source 105 is stopped, the bonding adhesive layer 230 may flow through the plurality of openings 262 of the sealing adhesive layer while maintaining contact with the tissue surrounding the tissue site 202. The bonding adhesive layer 230 may fill any leak paths between the sealing adhesive layer 232 and the epidermis 204 surrounding the tissue site 202. The bonding adhesive layer 230 may hold the cover 125 in place at the tissue surrounding the tissue site 202 with the first bond strength, which may be greater than the second bond strength from the sealing adhesive layer 232. The first bond strength between the dressing 110 and the epidermis 204 surrounding the tissue site 202 may ensure that there is little to no pressure leaving the dressing 110 if the negative-pressure source 105 is actuated.

If the tissue surrounding the tissue site 202 is coupled to the bonding adhesive layer 230, the microsphere layer 226 may be pulled towards the stand-off layer 224. The plurality of microspheres 244 may be embedded into the adhesive 246 to eliminate the space 402. The microsphere layer 226 may then be pulled into the stand-off layer 224 to eliminate the space 406. Once the space 402 and the space 406 are eliminated, the pathway 247 may be eliminated and fluid can no longer flow between the stand-off layer 224 and the microsphere layer 226. Once the plurality of microspheres 244 are embedded into the adhesive 246, the microsphere layer 226 may have the second tack. The microsphere layer 226 may have the second tack because the plurality of microspheres 244 may be embedded into the adhesive 246 and a greater amount of the adhesive 246 may be exposed to the stand-off layer 224. When a greater amount of the adhesive 246 is exposed to the stand-off layer 224, the second tack coupling the microsphere layer 226 and the stand-off layer 224 may be greater than the first tack and may adhesively couple the microsphere layer 226 and the stand-off layer 224 together. Once the pathway 247 has been eliminated, the negative-pressure source 105 may be actuated to the second predetermined pressure.

In alternate embodiments, where the plurality of microspheres 244 are disposed on the second surface 242 of the microsphere layer 226, the microsphere layer 226 may be pulled into the first chamber 272 with the stand-off layer 224 when the negative-pressure source 105 is operated at the first predetermined pressure such that both the stand-off layer 224 and the microsphere layer 226 are pierced by the first piercing element 280. Then, negative pressure from the negative-pressure source 105 may be manifolded between the microsphere layer 226 and the film layer 228. The tissue surrounding the tissue site 202 may be adhered to the bonding adhesive layer 230 and the plurality of microspheres 244 of the microsphere layer 226 may be embedded into the adhesive 246 substantially as described above. Once the plurality of microspheres 244 are embedded into the adhesive 246, the microsphere layer 226 may be adhered to the stand-off layer 224 and to the film layer 228 with the second tack and any pathways between the stand-off layer 224, the microsphere layer 226, and the film layer 228 may be eliminated.

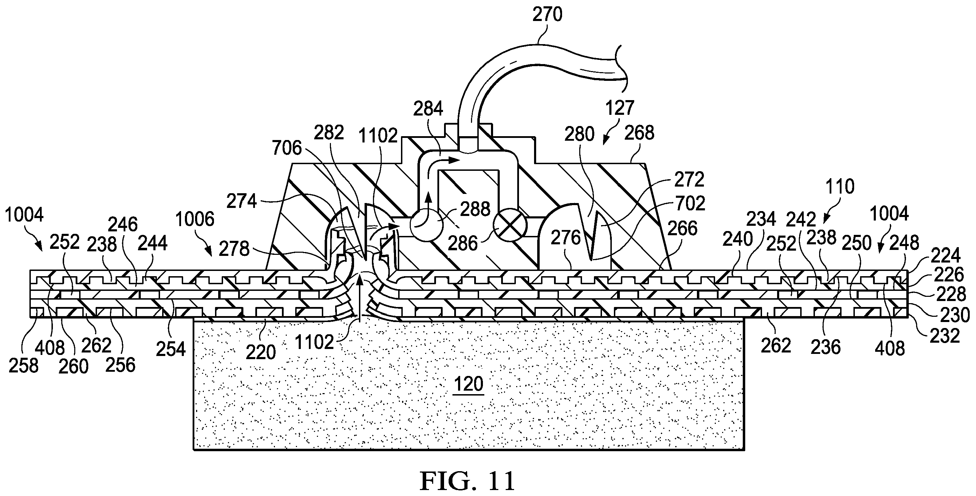

Figure 11 is a sectional view of the dressing 110 and the dressing interface 127 of the therapy system 100. After the cover 125 is adhered to the tissue site 202 with the first bond strength and the stand-off layer 224 is adhered to the microsphere layer 226 with the second tack, the negative-pressure source 105 may begin operating at the second predetermined pressure. Once the negative-pressure source 105 is adjusted to the second predetermined pressure, the first valve 286 may close and the second valve 288 may open. In some embodiments, the second predetermined pressure may act on the dressing 110 with a stronger force than the first predetermined pressure. The first chamber 272 may no longer be in fluid communication with the negative-pressure source 105 and the second chamber 274 may be in fluid communication with the negative-pressure source 105. Once the second valve 288 is open, the negative-pressure source 105 may generate a fluid flow 1102 from the second chamber 274.

The cover 125 may be drawn into the second chamber 274 as the fluid flow 1102 is drawn from the second chamber 274. The second piercing end 706 of the second piercing element 282 may pierce through the stand-off layer 224, the microsphere layer 226, the film layer 228, the bonding adhesive layer 230, and the sealing adhesive layer 232. The second piercing end 706 of the second piercing element 282 may then pierce through the film 220 of the tissue interface 120. Once the film 220 is pierced, the negative-pressure source 105 may be in fluid communication with the tissue interface 120. The therapy system 100 may then apply negative pressure from the negative-pressure source 105 across the tissue site 202 through the tissue interface 120 in an effort to treat the tissue site 202 with negative pressure wound therapy.

The systems, apparatuses, and methods described herein may provide significant advantages. For example, the therapy system 100 may help to create a better seal between the dressing 110 and the epidermis 204 surrounding the tissue site 202 by providing negative-pressure directly to the periphery 1004 of the cover 125 before applying negative-pressure to the tissue site 202. The therapy system 100 may also reduce the amount of time that it takes a clinician to apply the dressing 110 to the tissue site 202 because it can easily be picked up and moved prior to negative pressure being applied to the periphery 1004 of the cover 125. The therapy system 100 may also reduce leakage, which may increase the total battery life of the therapy system 100 and may provide a better quality product, which may increase patient comfort.

While shown in a few illustrative embodiments, a person having ordinary skill in the art will recognize that the systems, apparatuses, and methods described herein are susceptible to various changes and modifications that fall within the scope of the appended claims. Moreover, descriptions of various alternatives using terms such as "or" do not require mutual exclusivity unless clearly required by the context, and the indefinite articles "a" or "an" do not limit the subject to a single instance unless clearly required by the context. Components may also be combined or eliminated in various configurations for purposes of sale, manufacture, assembly, or use. For example, in some configurations the dressing 110, the container 115, or both may be eliminated or separated from other components for manufacture or sale. In other example configurations, the controller 130 may also be manufactured, configured, assembled, or sold independently of other components.

The appended claims set forth novel and inventive aspects of the subject matter described above, but the claims may also encompass additional subject matter not specifically recited in detail. For example, certain features, elements, or aspects may be omitted from the claims if not necessary to distinguish the novel and inventive features from what is already known to a person having ordinary skill in the art.

## Claims

1. A cover (125) for a dressing, the cover (125) comprising:
a stand-off layer (224) comprising a first surface (234) and a second surface (236) opposite the first surface (234), the second surface (236) comprising a plurality of surface features (238), wherein the plurality of surface features (238) comprises a plurality of projections extending away from the first surface (234) of the stand-off layer (224);
a microsphere layer (226) comprising a first surface (240) adjacent to the second surface (236) of the stand-off layer (224) and a second surface (242) opposite the first surface (240), the microsphere layer having a first tack and a second tack, the microsphere layer (226) further comprising a plurality of microspheres (244) and an adhesive (246) that provides the first tack to the first surface (240) of the microsphere layer (226) and provides the second tack to the first surface (240) of the microsphere layer (226) after a force has been applied to the cover (125), the microspheres (244) being embedded into the adhesive (246) when the force is applied to the cover (125), wherein the microsphere layer (226) and the plurality of surface features (238) manifold fluid across the cover (125);
a film layer (228) comprising a first surface (248) adjacent to the second surface (242) of the microsphere layer (226), and a second surface (250) opposite the first surface (248), wherein the film layer (228) is adhered to the microsphere layer (226) by the second tack of the adhesive (246);
a bonding adhesive layer (230) comprising a first surface (254) adjacent to the second surface (250) of the film layer (228) and a second surface (256) opposite the first surface (254); and
a sealing adhesive layer (232) comprising a plurality of openings (262), a first surface (258) adjacent to the second surface (256) of the bonding adhesive layer (230), and a second surface (260) opposite the first surface (258), the sealing adhesive layer (232) having a bond strength that is less than a bond strength of the bonding adhesive layer (230).

2. The cover of claim 1, wherein the second surface (236) of the stand-off layer (224) and the first surface (240) of the microsphere layer (226) form a pathway, wherein the pathway manifolds fluid across the cover (125) and prevents fluid flow in response to application of a force to the cover (125).

3. The cover of claim 1, wherein the first surface (240) of the microsphere layer (226) comprises the microspheres (244) and the second surface (242) of the microsphere layer (226) comprises the adhesive (246).

4. The cover of claim 1, wherein the film layer (228) comprises a plurality of perforations (252), or wherein the bonding adhesive layer (230) comprises a patterned adhesive, or wherein the sealing adhesive layer (232) comprises a patterned adhesive.

5. The cover of claim 1, wherein the bonding adhesive layer fills the plurality of openings of the sealing adhesive layer when a force is applied to the cover.

6. A dressing interface (127) for coupling a negative-pressure source (105) to a dressing (110), the dressing interface (127) comprising:
a first chamber (272);
a first piercing element (280) disposed in the first chamber (272);
a second chamber (274); and
a second piercing element (282) disposed in the second chamber (274);
wherein the dressing interface (127) draws fluid from the first chamber (272) at a first predetermined pressure and draws fluid from the second chamber (274) at a second predetermined pressure.

7. The dressing interface of claim 6, further comprising:
a first valve (286) fluidly coupled to the first chamber (272), wherein the first valve (286) is closed at ambient pressure and actuated in response to the first predetermined pressure; and
a second valve (288) fluidly coupled to the second chamber (274), wherein the second valve (288) is closed at ambient pressure and actuated in response to the second predetermined pressure.

8. The dressing interface of claim 7, wherein:
the first valve (286) enables fluid communication between the negative-pressure source (105) and the first chamber (272) in response to a pressure at the first predetermined pressure; and
the second valve (288) enables fluid communication between the negative-pressure source (105) and the second chamber (274) in response to a pressure at the second predetermined pressure.

9. The dressing interface of claim 7, wherein: the first valve (286) and the second valve (288) are manually actuated, or the first valve (286) and the second valve (288) are communicatively coupled to a controller (130), wherein the controller (130) actuates the first valve (286) and the second valve (288).

10. The dressing interface of claim 6, further comprising a first surface (266) with a first opening (276) to the first chamber (272) and a second opening (278) to the second chamber (274), the first surface (266) being for coupling to the dressing, in particular wherein:
a piercing end (702) of the first piercing element (280) is a first distance from the first surface (266) of the dressing interface (127) and the first piercing element (280) is at a first angle relative to the first surface (266) of the dressing interface (127); and
a piercing end (706) of the second piercing element (282) is a second distance from the first surface (266) of the dressing interface (127) and the second piercing element (282) is at a second angle relative to the first surface (266) of the dressing interface (127).

11. A system for treating a tissue site with negative-pressure, the system comprising:
a dressing (110) comprising:
the cover (125) of any of claims 1 to 5; and
a tissue interface (120) comprising:
a first surface (216);
a second surface (218) opposite the first surface (216), the second surface (218) of the tissue interface (120) for positioning proximate to the tissue site; and
a film (220) coupled to the first surface (216) of the tissue interface (120);
and
the dressing interface (127) of any of claims 6 to 10.

12. The system of claim 11, further comprising a negative-pressure source (105) coupled to the dressing interface (127).

13. The system of claim 12, wherein the first chamber (272) is coupled to the negative-pressure source (105) with a first conduit (904) and the second chamber (274) is coupled to the negative-pressure source (105) with a second conduit (906), in particular wherein the negative-pressure source (105) is in fluid communication through the first conduit (904) to the first chamber (272) at the first predetermined pressure and is in fluid communication through the second conduit (906) to the second chamber (274) at the second predetermined pressure.

14. The system of claim 11, wherein the first piercing element (280) pierces the stand-off layer (224).

15. The system of claim 11, wherein the second piercing element (282) pierces the stand-off layer (224), the microsphere layer (226), the film layer (228), the bonding adhesive layer (230), the sealing adhesive layer (232), and the film (220).

## Patentansprüche

1. Eine Abdeckung (125) für einen Verband, die Abdeckung (125) aufweisend:
eine Abstandsschicht (224), aufweisend eine erste Oberfläche (234) und eine zweite Oberfläche (236) gegenüber der ersten Oberfläche (234), die zweite Oberfläche (236) aufweisend eine Mehrzahl von Oberflächenmerkmalen (238), wobei die Mehrzahl von Oberflächenmerkmalen (238) eine Mehrzahl von Vorsprüngen aufweist, die sich von der ersten Oberfläche (234) der Abstandsschicht (224) weg erstrecken;
eine Mikrosphärenschicht (226), aufweisend eine erste Oberfläche (240) benachbart zu der zweiten Oberfläche (236) der Abstandsschicht (224) und eine zweite Oberfläche (242) gegenüber der ersten Oberfläche (240), wobei die Mikrosphärenschicht eine erste Klebrigkeit und eine zweite Klebrigkeit aufweist, die Mikrosphärenschicht (226) ferner aufweisend eine Mehrzahl von Mikrosphären (244) und einen Klebstoff (246), der die erste Klebrigkeit an der ersten Oberfläche (240) der Mikrosphärenschicht (226) bereitstellt und die zweite Klebrigkeit an der ersten Oberfläche (240) der Mikrosphärenschicht (226) bereitstellt, nachdem eine Kraft auf die Abdeckung (125) ausgeübt wurde, wobei die Mikrosphären (244) in den Klebstoff (246) eingebettet werden, wenn die Kraft auf die Abdeckung (125) ausgeübt wird, wobei die Mikrosphärenschicht (226) und die Mehrzahl von Oberflächenmerkmalen (238) Fluid über die Abdeckung (125) verteilen;
eine Folienschicht (228), aufweisend eine erste Oberfläche (248) benachbart zu der zweiten Oberfläche (242) der Mikrosphärenschicht (226) und eine zweite Oberfläche (250) gegenüber der ersten Oberfläche (248), wobei die Folienschicht (228) an die Mikrosphärenschicht (226) durch die zweite Klebrigkeit des Klebstoffs (246) geklebt wird;
eine Bindungsklebeschicht (230), aufweisend eine erste Oberfläche (254) benachbart zu der zweiten Oberfläche (250) der Folienschicht (228) und eine zweite Oberfläche (256) gegenüber der ersten Oberfläche (254); und
eine abdichtende Klebeschicht (232), aufweisend eine Mehrzahl von Öffnungen (262), eine erste Oberfläche (258) benachbart zu der zweiten Oberfläche (256) der Bindungsklebeschicht (230) und eine zweite Oberfläche (260) gegenüber der ersten Oberfläche (258), wobei die abdichtende Klebeschicht (232) eine Bindungsstärke aufweist, die geringer als eine Bindungsstärke der Bindungsklebeschicht (230) ist.

2. Die Abdeckung nach Anspruch 1, wobei die zweite Oberfläche (236) der Abstandsschicht (224) und die erste Oberfläche (240) der Mikrosphärenschicht (226) einen Pfad ausbilden, wobei der Pfad Fluid über die Abdeckung (125) verteilt und einen Fluidfluss als Reaktion auf die Ausübung einer Kraft auf die Abdeckung (125) verhindert.

3. Die Abdeckung nach Anspruch 1, wobei die erste Oberfläche (240) der Mikrosphärenschicht (226) die Mikrosphären (244) aufweist und die zweite Oberfläche (242) der Mikrosphärenschicht (226) den Klebstoff (246) aufweist.

4. Die Abdeckung nach Anspruch 1, wobei die Folienschicht (228) eine Mehrzahl von Perforationen (252) aufweist, oder wobei die Bindungsklebeschicht (230) einen strukturierten Klebstoff aufweist, oder wobei die abdichtende Klebeschicht (232) einen strukturierten Klebstoff aufweist.

5. Die Abdeckung nach Anspruch 1, wobei die Bindungsklebeschicht die Mehrzahl von Öffnungen der abdichtenden Klebeschicht füllt, wenn eine Kraft auf die Abdeckung ausgeübt wird.

6. Eine Verbandschnittstelle (127) zum Ankoppeln einer Unterdruckquelle (105) mit einem Verband (110), die Verbandschnittstelle (127) aufweisend:
eine erste Fluidkammer (272);
ein erstes Durchstoßelement (280), das in der ersten Kammer (272) angeordnet ist;
eine Fluidkammer (274); und
ein zweites Durchstoßelement (282), das in der zweiten Kammer (274) angeordnet ist;
wobei die Verbandschnittstelle (127) Fluid aus der ersten Kammer (272) bei einem ersten vorbestimmten Druck ansaugt und Fluid aus der zweiten Kammer (274) bei einem zweiten vorbestimmten Druck ansaugt.

7. Die Verbandschnittstelle nach Anspruch 6, ferner aufweisend:
ein erstes Ventil (286), das an die erste Kammer (272) fluidisch gekoppelt ist, wobei das erste Ventil (286) bei Umgebungsdruck geschlossen ist und als Reaktion auf den ersten vorbestimmten Druck betätigt wird; und
ein zweites Ventil (288), das an die zweite Kammer (274) fluidisch gekoppelt ist, wobei das zweite Ventil (288) bei Umgebungsdruck geschlossen ist und als Reaktion auf den zweiten vorbestimmten Druck betätigt wird.

8. Die Verbandschnittstelle nach Anspruch 7, wobei:
das erste Ventil (286) eine Fluidverbindung zwischen der Unterdruckquelle (105) und der ersten Kammer (272) als Reaktion auf einen Druck zu dem ersten vorbestimmten Druck ermöglicht; und
das zweite Ventil (288) eine Fluidverbindung zwischen der Unterdruckquelle (105) und der zweiten Kammer (274) als Reaktion auf einen Druck bei dem zweiten vorbestimmten Druck ermöglicht.

9. Die Verbandschnittstelle nach Anspruch 7, wobei: das erste Ventil (286) und das zweite Ventil (288) manuell betätigt werden, oder das erste Ventil (286) und das zweite Ventil (288) an eine Steuerung (130) kommunikativ gekoppelt sind, wobei die Steuerung (130) das erste Ventil (286) und das zweite Ventil (288) betätigt.

10. Die Verbandschnittstelle nach Anspruch 6, ferner aufweisend eine erste Oberfläche (266) mit einer ersten Öffnung (276) zu der ersten Kammer (272) und einer zweiten Öffnung (278) zu der zweiten Kammer (274), wobei die erste Oberfläche (266) zum Ankoppeln mit dem Verband vorgesehen ist, insbesondere wobei:
ein durchstechendes Ende (702) des ersten durchstechenden Elements (280) in einem ersten Abstand von der ersten Oberfläche (266) der Verbandschnittstelle (127) ist und das erste durchstechende Element (280) in einem ersten Winkel relativ zu der ersten Oberfläche (266) der Verbandschnittstelle (127) ist; und
ein durchstechendes Ende (706) des zweiten durchstechenden Elements (282) in einem zweiten Abstand von der ersten Oberfläche (266) der Verbandschnittstelle (127) ist und das zweite durchstechende Element (282) in einem zweiten Winkel relativ zu der ersten Oberfläche (266) der Verbandschnittstelle (127) ist.

11. Ein System zum Behandeln einer Gewebestelle mit Unterdruck, das System aufweisend:
einen Verband (110), aufweisend:
die Abdeckung (125) nach einem der Ansprüche 1 bis 5; und
eine Gewebeschnittstelle (120), aufweisend:
eine erste Oberfläche (216);
eine zweite Oberfläche (218) gegenüber der ersten Oberfläche (216), wobei die zweite Oberfläche (218) der Gewebeschnittstelle (120) zum Positionieren in der Nähe der Gewebestelle vorgesehen ist; und
eine Folie (220), die an die erste Oberfläche (216) der Gewebeschnittstelle (120) gekoppelt ist; und
die Verbandschnittstelle (127) nach einem der Ansprüche 6 bis 10.

12. Das System nach Anspruch 11, ferner aufweisend eine Unterdruckquelle (105), die an die Verbandschnittstelle (127) gekoppelt ist.

13. Das System nach Anspruch 12, wobei die erste Kammer (272) an die Unterdruckquelle (105) mit einer ersten Leitung (904) gekoppelt ist und die zweite Kammer (274) an die Unterdruckquelle (105) mit einer zweiten Leitung (906) gekoppelt ist, insbesondere wobei die Unterdruckquelle (105) in Fluidverbindung durch die erste Leitung (904) mit der ersten Kammer (272) bei dem ersten vorbestimmten Druck steht und in Fluidverbindung durch die zweite Leitung (906) mit der zweiten Kammer (274) bei dem zweiten vorbestimmten Druck steht.

14. Das System nach Anspruch 11, wobei das erste Durchstoßelement (280) die Abstandsschicht (224) durchstößt.

15. Das System nach Anspruch 11, wobei das zweite Durchstoßelement (282) die Abstandsschicht (224), die Mikrosphärenschicht (226), die Folienschicht (228), die Bindungsklebeschicht (230), die abdichtende Klebeschicht (232) und die Folie (220) durchstößt.

## Revendications

1. Enveloppe (125) pour un pansement, l'enveloppe (125) comprenant :
une couche d'espacement (224) comprenant une première surface (234) et une seconde surface (236) opposée à la première surface (234), la seconde surface (236) comprenant une pluralité de caractéristiques de surface (238), dans laquelle la pluralité de caractéristiques de surface (238) comprend une pluralité de saillies s'étendant à l'écart de la première surface (234) de la couche d'espacement (224) ;
une couche de microsphères (226) comprenant une première surface (240) adjacente à la seconde surface (236) de la couche d'espacement (224) et une seconde surface (242) opposée à la première surface (240), la couche de microsphères ayant une première pégosité et une seconde pégosité, la couche de microsphères (226) comprenant en outre une pluralité de microsphères (244) et un adhésif (246) qui fournit la première pégosité à la première surface (240) de la couche de microsphères (226) et fournit la seconde pégosité à la première surface (240) de la couche de microsphères (226) après qu'une force a été appliquée à l'enveloppe (125), les microsphères (244) étant intégrées dans l'adhésif (246) lorsque la force est appliquée à l'enveloppe (125), dans laquelle la couche de microsphères (226) et la pluralité de caractéristiques de surface (238) collectent le fluide à travers l'enveloppe (125) ;
une couche de film (228) comprenant une première surface (248) adjacente à la seconde surface (242) de la couche de microsphères (226), et une seconde surface (250) opposée à la première surface (248), dans laquelle la couche de film (228) est adhérée à la couche de microsphères (226) par la seconde pégosité de l'adhésif (246) ;
une couche adhésive de liaison (230) comprenant une première surface (254) adjacente à la seconde surface (250) de la couche de film (228) et une seconde surface (256) opposée à la première surface (254) ; et
une couche adhésive d'étanchéité (232) comprenant une pluralité d'ouvertures (262), une première surface (258) adjacente à la seconde surface (256) de la couche adhésive de liaison (230), et une seconde surface (260) opposée à la première surface (258), la couche adhésive d'étanchéité (232) ayant une résistance de lien qui est inférieure à une résistance de lien de la couche adhésive de liaison (230).

2. Enveloppe selon la revendication 1, dans laquelle la seconde surface (236) de la couche d'espacement (224) et la première surface (240) de la couche de microsphères (226) forment une voie, dans laquelle la voie collecte le fluide à travers l'enveloppe (125) et empêche l'écoulement de fluide en réponse à l'application d'une force à l'enveloppe (125).

3. Enveloppe selon la revendication 1, dans laquelle la première surface (240) de la couche de microsphères (226) comprend les microsphères (244) et la seconde surface (242) de la couche de microsphères (226) comprend l'adhésif (246).

4. Enveloppe selon la revendication 1, dans laquelle la couche de film (228) comprend une pluralité de perforations (252), ou dans laquelle la couche adhésive de liaison (230) comprend un adhésif à motifs, ou dans laquelle la couche adhésive d'étanchéité (232) comprend un adhésif à motifs.

5. Enveloppe selon la revendication 1, dans laquelle la couche adhésive de liaison remplit la pluralité d'ouvertures de la couche adhésive d'étanchéité lorsqu'une force est appliquée à l'enveloppe.

6. Interface de pansement (127) permettant d'accoupler une source de pression négative (105) à un pansement (110), l'interface de pansement (127) comprenant :
une première chambre (272) ;
un premier élément de perçage (280) disposé dans la première chambre (272) ;
une seconde chambre (274) ; et
un second élément de perçage (282) disposé dans la seconde chambre (274) ;
dans laquelle l'interface de pansement (127) aspire le fluide de la première chambre (272) à une première pression prédéterminée et aspire le fluide de la seconde chambre (274) à une seconde pression prédéterminée.

7. Interface de pansement selon la revendication 6, comprenant en outre :
une première valve (286) accouplée fluidiquement à la première chambre (272), dans laquelle la première valve (286) est fermée à la pression ambiante et actionnée en réponse à la première pression prédéterminée ; et
une seconde valve (288) accouplée fluidiquement à la seconde chambre (274), dans laquelle la seconde valve (288) est fermée à la pression ambiante et actionnée en réponse à la seconde pression prédéterminée.

8. Interface de pansement selon la revendication 7, dans laquelle :
la première valve (286) permet une communication fluidique entre la source de pression négative (105) et la première chambre (272) en réponse à une pression à la première pression prédéterminée ; et
la seconde valve (288) permet une communication fluidique entre la source de pression négative (105) et la seconde chambre (274) en réponse à une pression à la seconde pression prédéterminée.

9. Interface de pansement selon la revendication 7, dans laquelle : la première valve (286) et la seconde valve (288) sont actionnées manuellement, ou la première valve (286) et la seconde valve (288) sont accouplées en communication à un dispositif de commande (130), dans laquelle le dispositif de commande (130) actionne la première valve (286) et la seconde valve (288).

10. Interface de pansement selon la revendication 6, comprenant en outre une première surface (266) avec une première ouverture (276) vers la première chambre (272) et une seconde ouverture (278) vers la seconde chambre (274), la première surface (266) étant destinée à l'accouplement avec le pansement, en particulier dans laquelle :
une extrémité de perçage (702) du premier élément de perçage (280) est à une première distance de la première surface (266) de l'interface de pansement (127) et le premier élément de perçage (280) est à un premier angle par rapport à la première surface (266) de l'interface de pansement (127) ; et
une extrémité de perçage (706) du second élément de perçage (282) est à une seconde distance de la première surface (266) de l'interface de pansement (127) et le second élément de perçage (282) est à un second angle par rapport à la première surface (266) de l'interface de pansement (127).

11. Système permettant de traiter un site tissulaire avec une pression négative, le système comprenant :
un pansement (110), comprenant :
l'enveloppe (125) selon l'une quelconque des revendications 1 à 5 ; et
une interface tissulaire (120) comprenant :
une première surface (216) ;
une seconde surface (218) opposée à la première surface (216), la seconde surface (218) de l'interface tissulaire (120) permettant de se positionner à proximité du site tissulaire ; et
un film (220) accouplé à la première surface (216) de l'interface tissulaire (120) ; et
l'interface de pansement (127) selon l'une quelconque des revendications 6 à 10.

12. Système selon la revendication 11, comprenant en outre une source de pression négative (105) accouplée à l'interface de pansement (127).

13. Système selon la revendication 12, dans lequel la première chambre (272) est accouplée à la source de pression négative (105) avec un premier conduit (904) et la seconde chambre (274) est accouplée à la source de pression négative (105) avec un second conduit (906), en particulier dans lequel la source de pression négative (105) est en communication fluidique par le biais du premier conduit (904) vers la première chambre (272) à la première pression prédéterminée et est en communication fluidique par le biais du second conduit (906) vers la seconde chambre (274) à la seconde pression prédéterminée.

14. Système selon la revendication 11, dans lequel le premier élément de perçage (280) perce la couche d'espacement (224).

15. Système selon la revendication 11, dans lequel le second élément de perçage (282) perce la couche d'espacement (224), la couche de microsphères (226), la couche de film (228), la couche adhésive de liaison (230), la couche adhésive d'étanchéité (232), et le film (220).
